# FASCICULE DE BREVET EUROPEEN

(11) **EP 1 517 727 B1**
(45) Date de publication et mention de la délivrance du brevet: **04.04.2007**
(21) Numéro de dépôt: 03762718.9
(22) Date de dépôt: 30.06.2003
(51) Int. Cl.: A61N 5/10, G21K 1/093, G21K 5/04

(54) **DISPOSITIF D'IRRADIATION D'UNE CIBLE PAR UN FAISCEAU DE HADRONS CHARGES, APPLICATION A LA HADRONTHERAPIE**
VORRICHTUNG ZUR BESTRAHLUNG EINES ZIELS MIT EINEM HADRON-GELADENEN STRAHL, VERWENDUNG IN DER HADRONTHERAPIE
DEVICE FOR IRRADIATING A TARGET WITH A HADRON-CHARGED BEAM, USE IN HADRONTHERAPY

(30) Priorité: 02.07.2002 FR 0208240; 23.07.2002 FR 0209330
(43) Date de publication de la demande: 30.03.2005
(73) Titulaire: COMMISSARIAT A L'ENERGIE ATOMIQUE, 75015 Paris (FR)
(72) Inventeur: MEOT, François, F-38000 GRENOBLE (FR)
(74) Mandataire: Poulin, Gérard
(86) Numéro de dépôt international: PCT/FR2003/002026
(87) Numéro de publication internationale: WO 2004/004832

(56) Documents cités:
- WO-A-00/40064
- WO-A-02/41948
- US-A- 4 962 317
- US-A- 5 039 867
- FUTAMI Y ET AL: "Broad-beam three-dimensional irradiation system for heavy-ion radiotherapy at HIMAC" NUCLEAR INSTRUMENTS & METHODS IN PHYSICS RESEARCH, SECTION - A: ACCELERATORS, SPECTROMETERS, DETECTORS AND ASSOCIATED EQUIPMENT, NORTH-HOLLAND PUBLISHING COMPANY. AMSTERDAM, NL, vol. 430, no. 1, 21 juin 1999 (1999-06-21), pages 143-153, XP004169951 ISSN: 0168-9002
- JONES W P ET AL: "Design of a beam transport system for a proton radiation therapy facility" PARTICLE ACCELERATOR CONFERENCE, 1999. PROCEEDINGS OF THE 1999 NEW YORK, NY, USA 27 MARCH-2 APRIL 1999, PISCATAWAY, NJ, USA,IEEE, US, 27 mars 1999 (1999-03-27), pages 2519-2521, XP010349299 ISBN: 0-7803-5573-3 cité dans la demande
- MEOT F ET AL: "Principles of the non-linear tuning of beam expanders" NUCLEAR INSTRUMENTS & METHODS IN PHYSICS RESEARCH, SECTION - A: ACCELERATORS, SPECTROMETERS, DETECTORS AND ASSOCIATED EQUIPMENT, NORTH-HOLLAND PUBLISHING COMPANY. AMSTERDAM, NL, vol. 379, no. 2, 11 septembre 1996 (1996-09-11), pages 196-205, XP002235833 ISSN: 0168-9002 cité dans la demande

## Description

### DOMAINE TECHNIQUE

La présente invention concerne un dispositif d'irradiation d'une cible par un faisceau de hadrons chargés, plus simplement appelés "hadrons" par la suite.

Elle concerne en particulier un dispositif d'irradiation d'une zone du corps humain au cours d'une séance de hadronthérapie, cette zone étant occupée par une tumeur.

L'invention trouve des applications dans tout domaine qui nécessite l'irradiation d'une cible à laquelle on ne peut accéder ou qui est difficilement accessible.

Elle s'applique par exemple à l'irradiation de déchets radioactifs qui sont placés dans un conteneur hermétiquement fermé, en vue de transmuter ces déchets.

L'invention s'applique plus particulièrement à la hadronthérapie.

Elle est susceptible d'être implantée dans tous les centres de hadronthérapie et de remplacer les techniques que l'on utilise actuellement pour irradier les tumeurs par des faisceaux de hadrons.

### ETAT DE LA TECHNIQUE ANTERIEURE

La hadronthérapie est une méthode thérapeutique qui relève des principes de la radiothérapie, à ceci près qu'elle utilise des faisceaux de hadrons, alors que la radiothérapie utilise des photons ou des électrons.

En tant que hadrons, on utilise généralement des ions légers tels que, par exemple, H⁺, He²⁺ et C⁴⁺.

Des faisceaux de tels ions sont produits par une installation d'accélérateur, généralement un cyclotron dans le cas des protons et un synchrotron dans le cas des ions plus lourds.

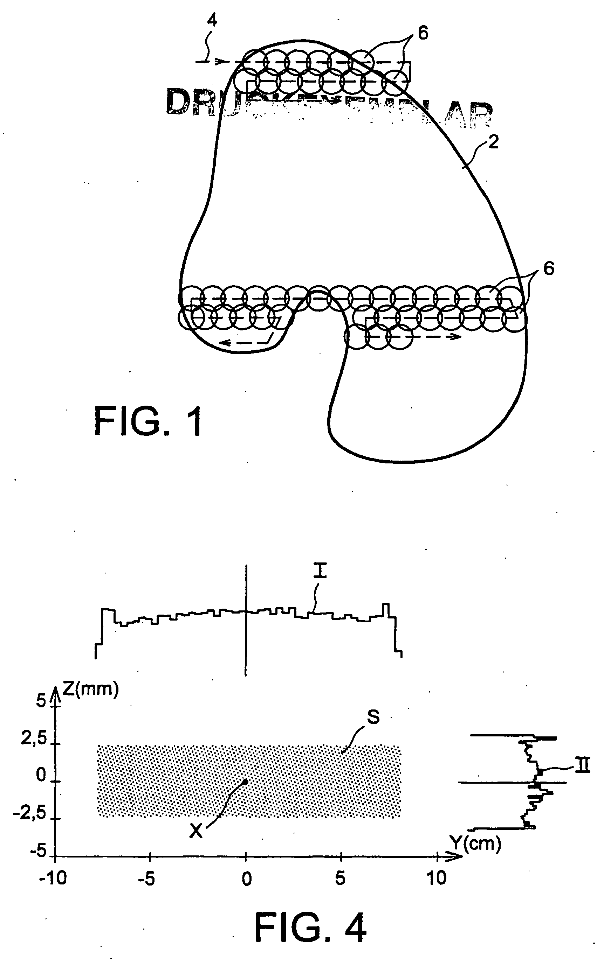

Des techniques de hadronthérapie sont connues par les documents suivants :
[1] "The medical accelerator HIMAC and the charged particle therapy in Japan", A. Kitagawa et F. Soga, Proc. PAC Conf. 2001, 18-22 juin 2001, Chicago, IL, USA
[2] "Proposal for a dedicated ion beam facility for cancer therapy", Eds. K.D. Gross, M. Pavlovic, GSI, Darmstadt, Septembre 1998.
   L'intérêt de la hadronthérapie par rapport à la radiothérapie réside dans l'existence du phénomène physique du "pic de Bragg" selon lequel les hadrons perdent la quasi-totalité de leur énergie cinétique à une profondeur définie dans le corps humain, causant ainsi un pic de dépôt de dose à cet endroit, que l'on appelle "pic de Bragg".
   La profondeur du pic de Bragg est fonction de l'énergie cinétique du faisceau incident et peut donc être réglée, typiquement, de un à quelques dizaines de centimètres, en accélérant les hadrons à des énergies cinétiques allant typiquement de 50 MeV/u à 450 MeV/u, au moyen d'un synchrotron, ou bien en utilisant un système d'analyse en moment dans le cas d'un faisceau de protons, que l'on accélère avec un cyclotron.
   Au sujet de ce phénomène du pic de Bragg, on se reportera par exemple au document [2].
   L'intérêt des ions légers et en particulier des ions de carbone par rapport aux protons (H⁺) réside dans leur meilleure efficacité biologique, qui crée une amplification du pic de Bragg.
   L'invention est profitable aux deux techniques de production de faisceaux de hadrons utilisant un accélérateur du type synchrotron ou cyclotron. Elle est susceptible de remplacer les systèmes d'irradiation utilisés dans les centres d'hadronthérapie, systèmes qui sont fondés sur l'un quelconque de ces deux types d'accélérateurs.
   Revenons sur les dispositifs connus, permettant l'irradiation d'une tumeur par un faisceau de hadrons.
   Les dimensions d'une tumeur peuvent atteindre plusieurs centimètres. Dans les installations existantes, l'irradiation du volume de la tumeur dans son ensemble est assurée de deux façons différentes.
   La première technique en usage fut l'irradiation passive, qui a toujours cours aujourd'hui car elle a le mérite d'être maîtrisée et bien connue.
   Une seconde technique, qui est aujourd'hui en cours de développement, est celle du balayage par pixels, ou balayage actif, qui permet une irradiation tri-dimensionnelle se conformant aux contours de la tumeur. C'est pourquoi on l'appelle "technique d'irradiation conformationnelle 3D".
   La technique d'irradiation passive est plutôt utilisée dans le cas des cyclotrons qui sont des machines à énergie fixe. Néanmoins, elle est également mise en oeuvre auprès de synchrotrons (voir le document [1]).
   Dans cette seconde technique, l'étalement de l'irradiation en profondeur est assuré par étalement du pic de Bragg, au moyen d'un matériau diffusant ("scattering material") qui induit une grande dispersion de l'énergie cinétique du faisceau de hadrons incident. Elle a été mise en oeuvre à la suite de travaux expérimentaux, visant à uniformiser des faisceaux par des moyens non-linéaires d'optique corpusculaire.
   A ce sujet, on consultera le document suivant :
[3] "Generation of rectangular beam distributions", B. Blind, Report MS H811, LANL, NM 87545
   La modélisation mathématique de cette technique a été faite par F. Méot et T. Aniel.
   A ce sujet on consultera les documents suivants :
[4] "On beam uniformization by non-linear optics" F. Méot et T. Aniel, Rapport du Laboratoire National Saturne, Réf CEA/DSM/GECA/GT/95-05, juillet 1995, pages 1 à 20.
[5] "Principles of the non-linear tuning of beam expanders", F. Méot et T. Aniel, Nuclear Instruments and Methods in Physics Research A379, 1996, pages 197 à 205.
   Dans le cas de l'irradiation passive, l'irradiation transversale de la tumeur est obtenue, quant à elle, par expansion transverse du faisceau, par exemple au moyen d'un système de balayage, appelé "wobulateur", qui étend le faisceau sur toute la largeur de la tumeur, en associant ce système de balayage à des matériaux diffusants, dont le rôle est d'uniformiser la densité transverse, ainsi qu'à des systèmes de diaphragmes, par exemple des collimateurs multilames, dont le rôle est de définir le mieux possible les contours de la zone à irradier.
   La technique d'irradiation passive présente au moins trois inconvénients importants.
   En effet, elle est fondée sur le principe de dégradation du faisceau incident : à partir d'un faisceau de grande qualité et quasiment mono-énergétique on forme un faisceau dont les dimensions sont étendues et l'énergie dispersée.
   De plus, cette technique conduit à intercaler, dans le faisceau incident, des éléments d'interception, appelés "matériaux dégradeurs".
   En outre, cette technique présente le défaut majeur de ne pas permettre une irradiation conformationnelle 3D précise : des zones externes à la tumeur sont inévitablement irradiées.
   L'utilisation de la technique de balayage actif est, quant à elle, préférentiellement envisagée dans les installations fondées sur un synchrotron, qui est une machine à énergie variable.
   Cependant, l'intérêt évident de cette technique fait qu'elle est maintenant également développée auprès de certaines installations à cyclotron, qui étaient jusqu'à présent limitées à l'irradiation passive, en utilisant un système de dispersion et d'analyse en moment qui permet d'induire une gamme d'énergie étendue.
   Dans cette technique de balayage actif, on utilise un faisceau de hadrons fin, dont le diamètre vaut typiquement quelques millimètres et l'irradiation en profondeur est assurée par sections, en ajustant l'énergie du faisceau de hadrons, ce qui a pour effet de déterminer la profondeur du pic de Bragg. Rappelons en effet qu'à une énergie des hadrons correspond une profondeur d'irradiation de quelques millimètres.
   L'irradiation transverse d'une section de la tumeur est assurée, quant à elle, par balayage de cette section au moyen du spot du faisceau, à la manière d'un faisceau de télévision, à une vitesse de l'ordre de 10 mètres par seconde.
   Une variante aujourd'hui préférée de cette technique (voir le document [2]) consiste à irradier la section de la tumeur pixel par pixel.
   Etant donné une profondeur et donc une section, le spot est maintenu sur un pixel, dont le diamètre est par conséquent celui du spot du faisceau, jusqu'à obtention de la dose requise, puis le spot est déplacé rapidement en translation jusqu'au pixel suivant, et ainsi de suite.
   Cette variante est schématiquement illustrée par la figure 1 où l'on voit l'irradiation d'une section de tumeur 2 par un faisceau de hadrons non représenté, qui est perpendiculaire au plan de la figure. Le balayage transverse de cette section est représenté par les pointillés 4 et l'on irradie successivement les pixels 6.
   Par rapport à l'irradiation passive, l'irradiation active, ou balayage actif, a l'avantage capital de permettre une conformation 3D et un réglage plus précis de la dose en tout point du volume à irradier, point que l'on appelle aussi "pixel volumique" ("volume pixel") ou voxel.
   Une autre technique d'expansion et d'uniformisation transverses en irradiation passive a récemment été proposée dans le domaine de la hadronthérapie.
   A ce sujet, on consultera le document suivant :
[6] "Design of a beam transport system for a proton radiation therapy facility", W.P Jones et G.P Berg, Proc. Particle Accelerator Conf., New-York, 1999, pages 2519-2521.
   Le mérite de cette autre technique est de ne pas intercepter le faisceau d'irradiation car elle est fondée sur un système uniquement composé de dispositifs d'optique corpusculaire : ce système utilise des lentilles magnétiques non-linéaires, chaque lentille étant un octupôle ou l'association d'un octupôle et d'un dodécapôle.
   Il convient de noter que cette technique avait déjà été proposée pour la production de faisceaux à distribution d'intensité transverse de grande étendue (de l'ordre du mètre-carré) et uniforme, pour le retraitement des déchets nucléaires par irradiation.
   A ce sujet, on consultera le document [3] précédemment cité.
   On évitait ainsi l'utilisation de matériaux diffusants qui ont le désavantage de tendre à détériorer le faisceau, car ils engendrent des queues de diffusion ("scattering tails") latérale et distale.
   La tendance actuelle est d'utiliser la technique du balayage par pixel, parce que c'est la seule qui permette une irradiation conformationnelle tridimensionnelle, sachant en outre que l'irradiation passive se trouve encore en usage principalement pour des raisons historiques.
   Le contrôle de la profondeur du pixel par le biais de l'énergie du faisceau incident est alors réalisé soit au moyen de l'accélérateur, à condition qu'il s'agisse d'un synchroton, soit par dégradation au moyen de matériaux d'interception.
   A ce sujet, on consultera les documents suivants :
[8] "Spot scanning irradiation with ¹¹C beams at Himac", E. Urakabe, FFAG-O2 Workshop, KEK, Tsukuba, 13-15 février 2002.
[9] "Flexible computational model of pencil beam dose distribution for spot-scanning", A. Molodojentsev et T. Sakae, FFAG-02 Workshop, KEK, Tsukuba, 13-15 février 2002.
   Dans le cas du balayage actif, la tendance actuelle se limite à oeuvrer au perfectionnement de la technique du balayage par pixel.
   On connaît en outre une technique de balayage comparable à celle qui est décrite dans le document [1], par le document suivant :
[10] " Accelerator facility PATRO for hadrontherapy at Hyogo Ion Beam Medical Center", A. Itano.

### EXPOSÉ DE L'INVENTION

La présente invention a pour but d'irradier la cible de manière mieux contrôlée que dans l'art antérieur mentionné plus haut, en délivrant les ions dans le volume exact de cette cible.

En particulier, l'invention a pour but d'améliorer considérablement la technique de balayage actif qui est mentionnée plus haut.

De façon précise, la présente invention a pour objet un dispositif d'irradiation d'une cible, notamment d'une zone du corps humain, conformément à la revendication 1.

On connaît aussi, par US 4962317, des moyens de manipulation d'un faisceau de particules chargées ; ces moyens engendrent un champ magnétique tel que le faisceau balaye une zone en forme de ruban sur une cible.

Selon un mode de réalisation préféré du dispositif objet de l'invention, les moyens d'optique corpusculaire comprennent au moins une lentille non-linéaire d'optique corpusculaire.

Ces moyens d'optique corpusculaire peuvent comprendre deux lentilles non-linéaires d'optique corpusculaire, prévues pour uniformiser la densité transversale du faisceau de hadrons chargés, suivant deux directions perpendiculaires l'une à l'autre et à la trajectoire de ce faisceau de hadrons chargés.

De préférence, chaque lentille non-linéaire d'optique corpusculaire est 2n-polaire, n étant un entier au moins égal à 4.

De préférence, les moyens de balayage comprennent une paire de dipôles magnétiques.

Selon un premier mode de réalisation particulier du dispositif objet de l'invention, les moyens de génération de faisceau de hadrons chargés comprennent un synchrotron et les moyens de réglage de l'énergie des hadrons chargés engendrés sont les moyens de réglage de l'énergie des hadrons chargés produits par ce synchrotron.

Selon un deuxième mode de réalisation particulier du dispositif objet de l'invention, les moyens de génération de faisceau de hadrons chargés comprennent un cyclotron et les moyens de réglage de l'énergie des hadrons chargés engendrés comprennent des moyens d'analyse en moment.

Dans un mode de réalisation particulier de l'invention, les moyens d'optique corpusculaire sont aptes à faire varier l'uniformisation de la densité transversale du faisceau de hadrons chargés suivant la longueur et/ou la largeur de la bande étroite.

Dans un mode de réalisation avantageux de l'invention, les moyens de balayage sont aptes à faire balayer la cible par le faisceau de hadrons chargés, à des profondeurs prédéfinies de cette cible et une pluralité de fois pour chacune de ces profondeurs, la dose délivrée à la cible à chaque fois étant égale à la dose totale prévue pour cette profondeur, divisée par le nombre de fois.

Les hadrons chargés utilisés dans la présente invention sont de préférence des ions légers ou plus exactement des noyaux légers, c'est-à-dire des noyaux dont le numéro atomique est inférieur typiquement à 20.

On choisit par exemple ces noyaux parmi H⁺, He⁺⁺ et C⁴⁺.

### BRÈVE DESCRIPTION DES DESSINS

La présente invention sera mieux comprise à la lecture de la description d'exemples de réalisation donnés ci-après, à titre purement indicatif et nullement limitatif, en faisant référence aux dessins annexés, sur lesquels:
- la figure 1 illustre schématiquement l'irradiation d'une section de tumeur par une technique connue de balayage transverse par pixel et a déjà été décrite,
- la figure 2 est une vue schématique d'un mode de réalisation particulier du dispositif objet de l'invention,
- la figure 3 est une vue schématique et partielle d'une variante du dispositif de la figure 2,
- la figure 4 est une vue en coupe transversale d'un faisceau d'irradiation qui est utilisable dans l'invention,
- la figure 5 illustre schématiquement l'irradiation d'une section de tumeur par un balayage uniformisé, lent et bidimensionnel de cette section, conformément à l'invention, et
- la figure 6 illustre schématiquement une juxtaposition de pixels pour l'uniformisation d'un dépôt de dose, conformément à une technique connue d'irradiation.

### EXPOSÉ DÉTAILLÉ DE MODES DE RÉALISATION PARTICULIERS

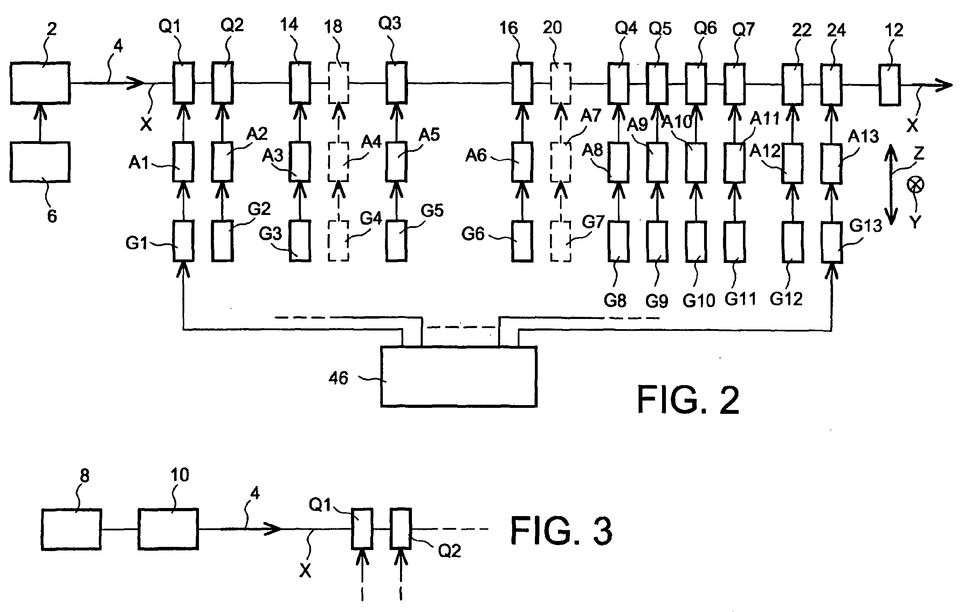

Un mode de réalisation particulier du dispositif objet de l'invention est schématiquement représenté sur la figure 2.

Ce dispositif est destiné à agir sur un faisceau de hadrons chargés, dont la trajectoire s'étend suivant un axe X.

On définit également deux directions Y et Z qui sont perpendiculaires l'une à l'autre ainsi qu'à l'axe X. A titre d'exemple, l'axe X est horizontal, la direction Y l'est aussi et la direction Z est verticale.

Dans l'exemple de la figure 2, les moyens 2 de génération du faisceau de hadrons 4 sont constitués par un synchrotron qui est pourvu de moyens 6 permettant de régler l'énergie des hadrons. Ces derniers sont par exemple des noyaux He²⁺ ou C⁴⁺.

Dans une variante qui est schématiquement et partiellement illustrée par la figure 3, les hadrons sont des protons (H⁺) et les moyens de génération du faisceau de protons 4 sont constitués par un cyclotron 8.

Ce dernier est suivi de moyens d'analyse en moment 10 qui permettent de régler l'énergie des protons.

On voit également la cible 12, par exemple une tumeur, que l'on veut irradier par le faisceau de hadrons 4.

Le dispositif conforme à l'invention, qui est représenté sur la figure 2, est installé entre le synchrotron 2 et la cible 12 (et entre les moyens d'analyse en moment 10 et cette cible dans le cas de la figure 3).

Ce dispositif comprend, le long de l'axe X, au moins une lentille non-linéaire d'uniformisation, au moins un dipôle magnétique de balayage et un ensemble de lentilles de focalisation.

Dans l'exemple, chaque lentille non-linéaire d'uniformisation est une lentille octupolaire et l'on en utilise deux, qui ont les références 14 et 16.

La lentille octupolaire 14 (respectivement 16) est destinée à uniformiser la densité transversale du faisceau de hadrons 4 suivant la direction horizontale Y (respectivement la direction verticale Z) .

On peut adjoindre une lentille dodécapolaire 18 (respectivement 20) à la lentille octupolaire 14 (respectivement 16).

Dans l'exemple, on utilise deux dipôles magnétiques ou aimants dipolaires magnétiques, qui ont les références 22 et 24.

On les place, de préférence, à moins de 10 m de la cible 12.

Le dipôle magnétique 22 (respectivement 24) est destiné à faire balayer la cible 12 par le faisceau de hadrons 4 suivant la direction horizontale Y (respectivement la direction verticale Z).

Dans l'exemple, les lentilles de focalisation sont des lentilles quadrupolaires, qui sont respectivement numérotées de Q1 à Q7.

Dans cet exemple, on trouve successivement : la lentille Q1 (divergente), la lentille Q2 (convergente), la lentille 14, l'éventuelle lentille 18, la lentille Q3 (divergente), la lentille 16, l'éventuelle lentille 20, la lentille Q4 (convergente), la lentille Q5 (divergente), la lentille Q6 (convergente), la lentille Q7 (divergente), le dipôle 22 et le dipôle 24.

Le dispositif de la figure 2 se classe dans la catégorie des dispositifs d'irradiation active. Il préserve donc l'avantage capital de la conformation 3D précise, tout en lui apportant des améliorations majeures. Il en est de même pour la variante de la figure 3.

Ce dispositif s'apparente aux dispositifs de balayage actif tels qu'on les a décrits plus haut, en ce que l'irradiation par le faisceau 4 est effectivement contrôlée dans les trois dimensions de l'espace, au moyen des dipôles de balayage 22 et 24, que l'on place quelques mètres en amont de la tumeur, ainsi qu'au moyen du réglage en énergie du faisceau incident 4.

Pour ce qui concerne la distribution transverse du faisceau, précisons que la zone transversale irradiée est étendue, en une bande étroite, de densité uniforme dans l'une ou bien dans les deux directions Y et Z, et de longueur en principe égale à la largeur locale de la section de tumeur en cours d'irradiation.

Dans l'invention, on utilise un dispositif d'optique corpusculaire, qui n'intercepte donc pas le faisceau de hadrons. Il comprend une ou deux lentilles non-linéaires.

Dans l'exemple de la figure 2, on utilise les deux lentilles non-linéaires 14 et 16, appelées lentilles d'uniformisation, intégrées à la ligne optique qui transporte le faisceau de hadrons vers un patient dont on irradie la tumeur.

On utilise en outre un dispositif de balayage du faisceau qui comprend, dans l'exemple, les deux dipôles magnétiques 22 et 24. Ces derniers sont du genre de ceux qui sont utilisés pour le balayage actif.

Certes, on connaît déjà la technique d'uniformisation transverse, au moyen d'une ou deux lentilles non-linéaires (voir les documents [3] et [6] mentionnés plus haut), et la technique de balayage du faisceau, au moyen d'une paire de dipôles magnétiques.

Cependant, la présente invention combine ces deux techniques de façon tout à fait originale et avantageuse.

Revenons sur l'uniformisation transverse mise en oeuvre dans l'invention grâce à un dispositif non-linéaire d'optique corpusculaire.

Dans sa forme naturelle (à la sortie du synchrotron 2 ou des moyens d'analyse en moment 10), le faisceau 4 présente une densité transverse en forme de cloche (comme une courbe gaussienne) dans chacune des deux directions Y et Z, qui sont orthogonales à sa direction de propagation X.

Mais, dans l'invention, il faut que cette densité soit uniforme, au moins dans l'une des directions Y et Z, voire dans les deux.

Cette uniformisation s'obtient au moyen de lentilles non-linéaires, à raison d'une lentille par direction.

On utilise des lentilles 2n-polaires, dont l'ordre 2n est un entier pair suffisamment élevé pour obtenir l'uniformisation souhaitée, 2n étant de préférence égal à 8 (lentilles octupolaires) ou supérieur à 8 si cela est nécessaire.

Comme on l'a vu, on peut même utiliser des lentilles non-linéaires complexes, formées chacune par un couple de lentilles non-linéaires, à savoir une lentille 2n-polaire et une lentille 2m-polaire, avec m>n≥4, en choisissant par exemple n=4 et m=6.

La figure 2 montre une ligne optique qui assure une telle uniformisation, dans le plan XY et dans le plan XZ à titre d'exemple.

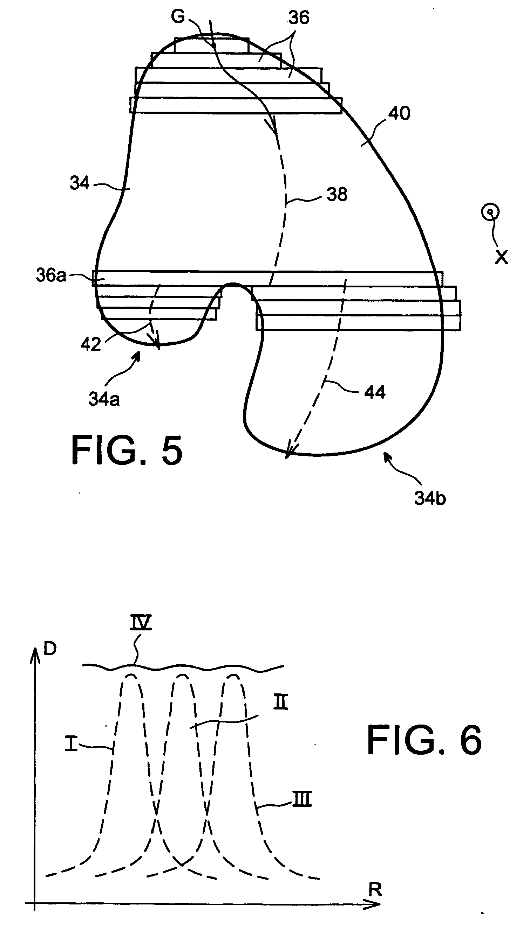

La section transversale S du faisceau 4, qui est perpendiculaire à l'axe X et forme une bande sensiblement rectangulaire (dont la longueur est parallèle à la direction Y et la largeur à la direction Z), ainsi que les profils du faisceau suivant Y (courbe I) et suivant Z (courbe II) qui en résultent, sont schématisés sur la figure 4 (donnée à titre purement indicatif et nullement limitatif, notamment en ce qui concerne les dimensions indiquées).

Dans le cas d'une ligne optique qui est conçue en vue de la mise en oeuvre de l'uniformisation selon l'invention, on prévoit les moyens d'optique corpusculaire de cette ligne en fonction de l'utilisation des lentilles non-linéaires.

Dans le cas de l'installation d'un dispositif conforme à l'invention sur une ligne optique existante, il peut suffire d'adapter les réglages optiques de focalisation pour tenir compte des impératifs liés au positionnement des lentilles non-linéaires ; éventuellement il peut s'avérer nécessaire de déplacer des quadrupôles de focalisation que comporte la ligne optique.

Revenons maintenant sur le balayage bidimensionnel.

Le dispositif de la figure 2 utilise un système de guidage lent du faisceau 4 (au sens où il est moins rapide que dans le cas du balayage actif), au moyen des deux aimants dipolaires 22 et 24, disposés par exemple juste en aval du dernier quadrupôle Q7 de la ligne optique de la figure 2, de sorte que l'empreinte du faisceau rectangulaire, telle qu'elle est schématisée sur la figure 4, balaye lentement la section de tumeur considérée, de la manière schématisée sur la figure 5.

Sur cette figure, la section de tumeur a la référence 34. Elle subit un balayage uniformisé, lent et bidimensionnel de la part du faisceau de hadrons, dont la section perpendiculairement à l'axe X a sensiblement la forme d'un rectangle 36 : le centre de gravité G de ce rectangle suit une ligne médiane 38 en même temps que le rectangle s'allonge ou se raccourcit pour suivre les contours 40 de la tumeur.

Il convient de noter que le rectangle glisse et que les contours de la zone ainsi balayée sont lisses, contrairement à ce que montre la figure 5.

De plus, dans l'exemple de cette figure 5, le bas de la tumeur se divise en deux parties 34a et 34b.

Dans ce cas, à partir d'un rectangle de balayage 36a, la ligne de balayage 38 est d'abord prolongée par une ligne de balayage 42, pour balayer l'une 34a de ces deux parties par des rectangles de balayage adaptés, puis on revient au rectangle 36a pour prolonger la ligne de balayage 38 par une autre ligne de balayage 44, en vue de balayer l'autre partie 34b par des rectangles de balayage adaptés.

La longueur du rectangle au point courant est ajustée, de façon très classique, au moyen de lentilles quadrupôlaires de focalisation (par exemple les quadrupôles Q4 à Q7). Sa largeur est, elle aussi, ajustable à loisir avec les mêmes quadrupôles. Il s'agit là de simples formations d'images sur le plan-image de la ligne optique, qui est le plan de la section de tumeur irradiée.

Cela nécessite un ajustement simultané de la (ou des deux) lentille(s) non-linéaire(s) car l'uniformisation dépend aussi du réglage des quadrupôles (voir le document [4]).

L'ensemble est réalisable de façon classique au moyen de générateurs de fonctions qui commandent les alimentations de ces diverses lentilles et sont eux-mêmes commandés par un logiciel.

Sur la figure 2, les références A1 à A13 désignent les alimentations qui sont respectivement associées aux composants Q1, Q2, 14, 18, Q3, 16, 20, Q4, Q5, Q6, Q7, 22 et 24.

Les références G1 à G13 désignent les générateurs de fonctions qui commandent respectivement ces alimentations A1 à A13 et la référence 46 désigne les moyens électroniques de traitement contenant le logiciel et prévus pour commander ces générateurs de fonctions.

Les données de ce logiciel résultent du planning de traitement du malade, élaboré au préalable par les médecins, au vu d'images obtenues par une ou plusieurs techniques d'imagerie connues de l'homme du métier.

On donne dans ce qui suit divers mérites et caractères innovants de l'invention.

Le balayage par rectangle uniformisé que l'on a décrit englobe le balayage par pixel car un pixel peut être considéré comme un petit rectangle.

Par ailleurs, le balayage par pixel est actuellement la technique préférée car il permet l'irradiation dite "conformationnelle 3D" : on peut régler le faisceau de particules en position et en énergie de façon à atteindre un pixel de position arbitraire dans le volume de la tumeur.

En outre, le balayage par pixel, avec arrêt sur chaque pixel, est préféré par rapport à un balayage continu de type télévision.

Pour démontrer les avantages que présente l'invention par rapport à toutes les autres techniques connues, il est donc suffisant de comparer les performances de l'invention à celles du seul balayage actif par pixel, qui constitue l'art antérieur le plus proche de l'invention.

### 1) Considérons d'abord la question des surintensités

Dans certaines conditions accidentelles, le faisceau de hadrons est susceptible de présenter un pic temporel d'intensité, lequel induit immédiatement l'arrêt de l'irradiation par des systèmes de sécurité appropriés (non représentés), connus de l'homme du métier.

Il en résulte une sur-irradiation plus ou moins prononcée de la zone visée par le faisceau à ce moment-là. C'est là un problème important en hadronthérapie, dont on cherche encore la solution optimale.

Dans le cas du dispositif conforme à l'invention, le rectangle d'irradiation a le plus souvent une surface très supérieure à celle qu'aurait un pixel considéré dans la technique de balayage actif. Pour s'en convaincre, il suffit de comparer les figures 1 et 5 qui montrent des sections de tumeur identiques.

Par conséquent, dans le cas de l'invention, illustré par la figure 5, le pic accidentel provoque une sur-irradiation surfacique plus faible que dans l'art antérieur illustré par la figure 1, le rapport des sur-irradiations surfaciques étant proportionnel au rapport des surfaces.

La nuisance est donc amoindrie, voire éliminée, sachant que l'irradiation met en jeu des effets non-linéaires présentant des phénomènes de seuil, tel que le seuil à 2 Gray (voir le document [1]).

### 2) Considérons ensuite la question de l'homogénéité de dose

A) - L'intensité du faisceau extrait de l'accélérateur doit être aussi constante que possible dans le temps (en exprimant par exemple cette intensité en nombre de particules par seconde). En réalité, cette intensité est sujette à des fluctuations, dans des gammes de fréquences qui dépendent par exemple des techniques d'extraction de l'accélérateur.
   Le dispositif de l'invention intègre ces fluctuations, c'est-à-dire les lisse, mieux que ne le fait la technique de balayage actif, parce que le balayage par rectangle s'effectue à vitesse plus faible : en quelque sorte, un rectangle équivalent à N pixels stationne sur une région donnée N fois plus longtemps que l'un quelconque de ces N pixels.
B) - Dans l'irradiation par pixel, l'uniformité de dose entre deux pixels juxtaposés d'une même ligne ou entre deux pixels de lignes successives, est assurée par une recouvrement présumé adéquat des distributions en cloche.

A ce sujet, on considèrera la figure 1 et la figure 6 qui illustre schématiquement cette juxtaposition des pixels en vue de l'uniformisation du dépôt de dose.

Les profils I, II et III de trois pixels juxtaposés sont tracés dans un repère où la position P est portée en abscisses et la dose déposée D en ordonnées. Le profil résultant a la référence IV.

Le résultat n'est jamais garanti et dépend en outre fortement du profil transverse de la dose dans chaque pixel, dont la distribution n'est pas nécessairement en forme de cloche, ni symétrique.

Le dispositif de l'invention fonctionne de manière très différente. Dans la direction du balayage, l'uniformité du dépôt de dose est assurée en régulant la vitesse du balayage par asservissement, au moyen d'instruments de mesure non représentés, identiques à ceux qui sont utilisés dans le cas du balayage actif.

Au sujet de ces instruments, on consultera les documents [1, 2] déjà cités

Dans la direction perpendiculaire au balayage, qui est la direction de la longueur du rectangle, l'uniformité est intrinsèque. Elle résulte de l'uniformisation par les lentilles non-linéaires.

### 3) Considérons en outre la question des tumeurs mobiles.

Le fait qu'une tumeur soit susceptible de bouger est l'une des difficultés de la hadronthérapie. Le balayage par rectangle est également avantageux par rapport au balayage par pixel, en cas de mouvement inopiné.

En effet, considérons le cas le plus défavorable où le mouvement de la tumeur éloigne la zone d'irradiation actuelle de la zone précédemment irradiée. Alors, dans le cas du balayage par pixel, toute une bande se trouve non-irradiée, tandis qu'il n'y a pas de lacune dans le cas du balayage par rectangle: toute la bande a été irradiée pendant l'intervalle de temps précédant le mouvement, aussi peu cela soit-il.

On donne dans ce qui suit des précisions complémentaires au sujet de l'invention.

On est libre de laisser, à un rectangle d'irradiation (voir les figures 4 et 5), un profil en cloche dans la direction du balayage selon Z. L'une des deux lentilles non-linéaires est alors inactive et l'uniformisation ne se fait que dans l'autre direction, à savoir celle de la longueur du rectangle.

L'uniformisation éventuelle dans la direction de la largeur du rectangle rend abrupts les bords de la distribution dans cette direction (voir les histogrammes de la figure 4), par rapport à une distribution en forme de cloche.

On est libre d'utiliser cette propriété pour une meilleure définition des contours de la zone irradiée.

Inversement, si ces bords apparaissent trop abrupts, il est possible de modifier leur pente, d'une manière contrôlée, en agissant sur la lentille non-linéaire adéquate.

On peut aussi imaginer d'allumer et d'éteindre cette lentille progressivement aux extrémités du balayage pour améliorer l'adaptation aux contours de la tumeur.

Dans les installations à cyclotron, on développe aujourd'hui la technique d'irradiation 3D conformationnelle (à laquelle se prêtent mieux les synchrotrons), au moyen de l'analyse en moment. On peut donc utiliser un dispositif conforme à l'invention dans de telles installations : il suffit de le placer en aval des moyens d'analyse en moment, comme on l'a déjà mentionné.

La durée de l'irradiation d'une section est en toute rigueur plus courte avec le balayage par rectangle uniformisé, qu'avec le balayage par pixel :
1/ on fait des économies de déplacement d'un pixel au suivant, et
2/ le balayage par rectangle est continu ; il ne présente aucune interruption sauf éventuellement à certains changements de concavité de la tumeur (comme sur la figure 5).

Le suivi des contours d'une section à irradier est une opération délicate. Le balayage uniforme en rectangle permet de mieux s'y adapter : on peut réduire la largeur du rectangle et augmenter la vitesse du balayage. Au contraire, pour un balayage par pixel, on ne peut pas réduire autant que l'on veut le diamètre du pixel, sinon on ne maîtrise plus l'uniformité entre pixels.

Dans l'invention, pour une profondeur donnée, il est possible de réaliser le balayage en plusieurs couches (c'est-à-dire en plusieurs fois), de façon éventuellement croisée.

Il suffit pour cela de diviser la dose totale nécessaire pour cette profondeur par le nombre de couches.

Dans ce cas, on facilite l'uniformisation de la dose en procédant par étapes, ce qui permet des ajustements, par exemple sur les bords de la tumeur. En outre, on augmente un peu plus les avantages déjà cités du balayage en rectangle:
- en ce qui concerne les surintensités, un pic temporel accidentel voit son intensité encore diminuée, et il en est donc de même pour la sur-irradiation qui s'ensuit ;
- en ce qui concerne l'homogénéité de dose, les fluctuations temporelles d'intensité, qui sont liées à l'extraction lente d'un synchrotron, sont encore plus lissées ;
- en ce qui concerne les tumeurs mobiles, si une tumeur bouge lors du balayage d'une couche, l'existence des couches suivantes ou précédentes limite à une seule couche le manque de dose.

## Revendications

1. Dispositif d'irradiation d'une cible (12), notamment d'une zone du corps humain, par un faisceau (4) de hadrons chargés, ce faisceau étant produit par des moyens de génération de faisceau de hadrons chargés, ce dispositif comprenant:
- des moyens d'optique corpusculaire (14, 16, 18, 20), prévus pour uniformiser la densité transversale du faisceau de hadrons chargés, suivant au moins une direction perpendiculaire à la trajectoire de ce faisceau de hadrons chargés, et
- des moyens (6, 22, 24; 10, 22, 24) de contrôle tridimensionnel de l'irradiation de la cible par ce faisceau de hadrons chargés,
les moyens de contrôle tridimensionnel comprenant:
- des moyens de réglage de l'énergie des hadrons chargés engendrés et
- des moyens de balayage, aptes à déplacer le faisceau de hadrons chargés pour lui faire balayer la cible par une bande étroite, sensiblement rectangulaire, et aptes à faire suivre sur une ligne médiane de la cible le centre de gravité de la bande étroite, tout en allongeant ou en raccourcissant cette bande étroite afin de suivre les contours de la cible.

2. Dispositif selon la revendication 1, dans lequel les moyens d'optique corpusculaire comprennent au moins une lentille non-linéaire d'optique corpusculaire.

3. Dispositif selon la revendication 1, dans lequel les moyens d'optique corpusculaire comprennent deux lentilles non-linéaires d'optique corpusculaire, prévues pour uniformiser la densité transversale du faisceau de hadrons chargés, suivant deux directions perpendiculaires l'une à l'autre et à la.trajectoire de ce faisceau de hadrons chargés.

4. Dispositif selon l'une quelconque des revendications 2 et 3, dans lequel chaque lentille non-linéaire d'optique corpusculaire est 2n-polaire, 2n étant un entier pair au moins égal à 8.

5. Dispositif selon la revendication 1, dans lequel les moyens de balayage comprennent une paire de dipôles magnétiques.

6. Dispositif selon l'une quelconque des revendications 1 à 5, dans lequel les moyens de génération de faisceau de hadrons chargés comprennent un synchrotron et les moyens de réglage de l'énergie des hadrons chargés engendrés sont les moyens de réglage de l'énergie des hadrons chargés produits par ce synchrotron.

7. Dispositif selon l'une quelconque des revendications 1 à 5, dans lequel les moyens de génération de faisceau de hadrons chargés comprennent un cyclotron et les moyens de réglage de l'énergie des hadrons chargés engendrés comprennent des moyens d'analyse en moment.

8. Dispositif selon l'une quelconque des revendications 1 à 7, dans lequel les moyens d'optique corpusculaire sont aptes à faire varier l'uniformisation de la densité transversale du faisceau de hadrons chargés suivant la longueur et/ou la largeur de la bande étroite.

9. Dispositif selon l'une quelconque des revendications 1 à 8, dans lequel les moyens de balayage sont aptes à faire balayer la cible par le faisceau de hadrons chargés, à des profondeurs prédéfinies de cette cible et une pluralité de fois pour chacune de ces profondeurs, la dose délivrée à la cible à chaque fois étant égale à la dose totale prévue pour cette profondeur, divisée par le nombre de fois.

10. Dispositif selon l'une quelconque des revendications 1 à 9, dans lequel les hadrons chargés sont des noyaux légers.

## Claims

1. Irradiation device for a target (12), particularly an area of the human body, by a charged hadron beam (4), this beam being produced by means of generating a charged hadron beam, this device comprising:
- corpuscular optics means (14, 16, 18, 20), designed to make the transverse density of the charged hadron beam uniform, along at least one direction perpendicular to the trajectory of this charged hadron beam, and
- means (6, 22, 24; 10, 22, 24) for the three-dimensional control of the irradiation of the target by this charged hadron beam, the three-dimensional control means including:
- means of adjusting the energy of the generated charged hadrons and
- scanning means capable of displacing the charged hadron beam to make it scan the target in a narrow substantially rectangular band, capable of causing the centre of gravity of the narrow band to follow on a median line of the target while lengthening or shortening this narrow band in order to follow the contours of the target.

2. Device according to claim 1, in which the corpuscular optics means comprise at least one non-linear corpuscular optics lens.

3. Device according to claim 1, in which the corpuscular optics means include two non-linear corpuscular optics lenses designed to make the transverse density of the charged hadron beam uniform, along two directions perpendicular to each other and to the trajectory of this charged hadron beam.

4. Device according to either claim 2 or 3, in which each non-linear corpuscular optics lens is 2n-polar, where 2n is an integer equal to at least 8.

5. Device according to claim 1, in which the scanning means include a pair of magnetic dipoles.

6. Device according to any one of the claims 1 to 5, in which the charged hadron beam generation means comprise a synchrotron and the means for adjusting the energy of the generated charged hadrons are the means of adjusting the energy of the charged hadrons produced by this synchrotron.

7. Device according to any one of the claims 1 to 5, in which the means of generation of the charged hadron beam comprise a cyclotron and the means of adjusting the energy of the generated charged hadrons include moment analysis means.

8. Device according to any one of claims 1 to 7, in which the corpuscular optics means are capable of varying the uniformization of the transverse density of the charged hadron beam depending on the length and/or the width of the narrow band.

9. Device according to any one of claims 1 to 8, in which the scanning means are capable of making the charged hadron beam scan the target at predetermined depths of this target, a plurality of times for each of these depths, the dose delivered to the target each time being equal to the total dose required for this depth, divided by the number of times.

10. Device according to any one of claims 1 to 9, in which the charged hadrons are light nuclei.

## Patentansprüche

1. Vorrichtung zur Bestrahlung eines Ziels (12), insbesondere eines Bereichs des menschlichen Körpers, mit einem Strahl (4) geladener Hadronen, wobei dieser Strahl durch Einrichtungen zur Erzeugung eines Strahls geladener Hadronen erzeugt wird und diese Vorrichtung dabei umfasst:
- Korpuskularoptik-Einrichtungen (14, 16, 18, 20), vorgesehen zur gleichmäßigen Verteilung der transversalen Dichte des Strahls geladener Hadronen entsprechend wenigstens einer Richtung senkrecht zu dem Weg dieses Strahls geladener Hadronen, und
- Einrichtungen (6, 22, 24; 10, 22, 24) zur dreidimensionalen Kontrolle der Bestrahlung des Ziels durch diesen Strahl geladener Hadronen, wobei diese Einrichtungen zur dreidimensionalen Kontrolle umfassen:
- Regelungseinrichtungen der Energie der erzeugten geladenen Hadronen, und
- Abtasteinrichtungen, fähig den Strahl geladener Hadronen zu verschieben, um ihn das Ziel mit einem schmalen im Wesentlichen rechteckigen Streifen abtasten zu lassen, und fähig, den Schwerpunkt des schmalen Streifens einer Mittellinie des Ziels folgen zu lassen und dabei diesen schmalen Streifen so zu verlängern oder zu verkürzen, dass er den Konturen des Ziels folgt.

2. Vorrichtung nach Anspruch 1, bei der die Korpuskularoptik-Einrichtungen wenigstens eine nichtlineare Korpuskularoptik-Linse umfassen.

3. Vorrichtung nach Anspruch 1, bei der die Korpuskularoptik-Einrichtungen zwei nichtlineare Korpuskularoptik-Linsen umfassen, dazu vorgesehen, die transversale Dichte des Strahls geladener Hadronen gleichmäßig zu verteilen, entsprechend zwei Richtungen senkrecht zueinander und zu dem Weg dieses Strahls geladener Hadronen.

4. Vorrichtung nach einem der Ansprüche 2 und 3, bei der jede nichtlineare Korpuskularoptik-Linse 2n-polar (2n-polaire) ist, wobei 2n eine ganze Zahl wenigstens gleich 8 ist.

5. Vorrichtung nach Anspruch 1, bei der die Abtasteinrichtungen ein Paar magnetischer Dipole umfassen.

6. Vorrichtung nach einem der Ansprüche 1 bis 5, bei der die Einrichtungen zur Erzeugung des Strahls geladener Hadronen ein Synchrotron umfassen und die Regelungseinrichtungen der Energie der erzeugten geladenen Hadronen Einrichtungen zur Regelung der Energie der durch dieses Synchrotron erzeugten geladenen Hadronen sind.

7. Vorrichtung nach einem der Ansprüche 1 bis 5, bei der die Einrichtungen zur Erzeugung des Strahls geladener Hadronen ein Zyklotron umfassen und die Regelungseinrichtungen der Energie der erzeugten geladenen Hadronen Momentanalyseeinrichtungen umfassen.

8. Vorrichtung nach einem der Ansprüche 1 bis 7, bei der die Korpuskularoptik-Einrichtungen fähig sind, die gleichmäßige Verteilung der transversalen Dichte des Strahls geladener Hadronen entsprechend der Länge und/oder der Breite des schmalen Steifens zu variieren.

9. Vorrichtung nach einem der Ansprüche 1 bis 7, bei der die Abtasteinrichtungen fähig sind, das Ziel mit dem Strahl geladener Hadronen in vordefinierten Tiefen dieses Ziels abzutasten, und dies für jede dieser Tiefen eine Vielzahl von Malen, wobei die dem Ziel jedes Mal gelieferte Dosis gleich der für diese Dosis vorgesehenen Gesamtdosis geteilt durch die Anzahl der Male ist.

10. Vorrichtung nach einem der Ansprüche 1 bis 9, bei der die geladenen Hadronen leichte Kerne sind.
